# EUROPÄISCHE PATENTANMELDUNG

(11) **EP 3 550 240 A1**
(43) Veröffentlichungstag der Anmeldung: **09.10.2019**
(21) Anmeldenummer: 18166161.2
(22) Anmeldetag: 06.04.2018
(51) Int. Cl.: F25J 3/02, C10G 70/04, C07C 7/09

(54) **VERFAHREN ZUR TRENNUNG EINES KOMPONENTENGEMISCHS UND TRENNEINRICHTUNG**

(71) Anmelder: Linde AG, 80331 München (DE)
(72) Erfinder: Höfel, Torben, 81543 München (DE); Tuat Pham, Duc, 82377 Penzberg (DE)
(74) Vertreter: m patent group

(57) **Zusammenfassung**

Es wird ein Verfahren zur Trennung eines Komponentengemischs, das im Wesentlichen Kohlenwasserstoffe mit zwei oder zwei und mehr Kohlenstoffatomen, Methan und Wasserstoff enthält, unter Verwendung einer Destillationseinrichtung (10) vorgeschlagen. Fluid (a, c, e, g, i) des Komponentengemischs wird auf einem ersten Druckniveau stufenweise abgekühlt, wobei jeweils erste Kondensate (b, d, f, h, j) aus dem Fluid (a, c, e, g, i) abgeschieden werden. Fluid (k) des Komponentengemischs, das hiernach gasförmig verbleibt, wird in einem Expander auf ein zweites Druckniveau entspannt, wobei ein zweites Kondensat (I) erhalten wird. Fluid der ersten Kondensate (b, d, f, h, j) wird von dem ersten Druckniveau auf das zweites Druckniveau entspannt und mit dem Fluid der zweiten Kondensate in die Destillationseinrichtung (10) eingespeist, die auf dem zweiten Druckniveau betrieben wird. Eine entsprechende Trenneinrichtung ist ebenfalls Gegenstand der vorliegenden Erfindung.

## Beschreibung

Die vorliegende Erfindung betrifft ein Verfahren zur Trennung eines Komponentengemischs, das im Wesentlichen Kohlenwasserstoffe mit zwei oder zwei und mehr Kohlenstoffatomen, Methan und Wasserstoff enthält, sowie eine Trenneinrichtung gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben. Durch Dampfspalten, aber auch unter Verwendung anderer Verfahren und Vorrichtungen, werden Komponentengemische in Form sogenannter Roh- oder Spaltgase enthalten, die nach einer Aufbereitung, die die Entfernung von Wasser und Kohlendioxid sowie von öl- oder benzinartigen Komponenten umfasst, zumindest teilweise in die jeweils enthaltenen Komponenten aufgetrennt werden. Dies kann auf unterschiedliche Weise erfolgen.

Die vorliegende Erfindung kann zur Entfernung von Methan und Wasserstoff aus einem Komponentengemisch, das im Wesentlichen Kohlenwasserstoffe mit zwei Kohlenstoffatomen, Methan und Wasserstoff enthält, aber arm an oder frei von schwereren Kohlenwasserstoffen ist, eingesetzt werden, und wird nachfolgend insbesondere unter Bezugnahme auf diese Variante beschrieben. Ein derartiges Komponentengemisch wird in den aus der zitierten Literatur bekannten Trennsequenzen insbesondere bei einer Deethanisierung des aufbereiteten Spaltgases erhalten, wenn diese Deethanisierung an erster Stelle des Trennprozesses steht. Wie unten nochmals erläutert, wird ein derartiges Komponentengemisch bzw. ein entsprechender Stoffstrom üblicherweise auch als "C2minus-Strom" bezeichnet.

Die vorliegende Erfindung kann jedoch auch zur Entfernung von Methan und Wasserstoff aus einem Komponentengemisch, das im Wesentlichen Kohlenwasserstoffe mit zwei und mehr Kohlenstoffatomen, Methan und Wasserstoff enthält, eingesetzt werden. Ein derartiges Komponentengemisch kann dabei insbesondere in Form des wie erläutert aufbereiteten, aber noch nicht weiter trenntechnisch behandelten Spaltgases vorliegen.

In beiden Fällen, also der Entfernung von Methan und Wasserstoff aus einem Komponentengemisch, das im Wesentlichen Kohlenwasserstoffe mit zwei Kohlenstoffatomen, Methan und Wasserstoff enthält, aber arm an oder frei von schwereren Kohlenwasserstoffen ist, und bei der Entfernung von Methan und Wasserstoff aus einem Komponentengemisch, das im Wesentlichen Kohlenwasserstoffe mit zwei und mehr Kohlenstoffatomen, Methan und Wasserstoff enthält, wird im Rahmen der vorliegenden Erfindung also eine sogenannte Demethanisierung vorgenommen. Diese steht im ersteren Fall an zweiter und im letzteren Fall an erster Stelle einer entsprechenden Trennsequenz.

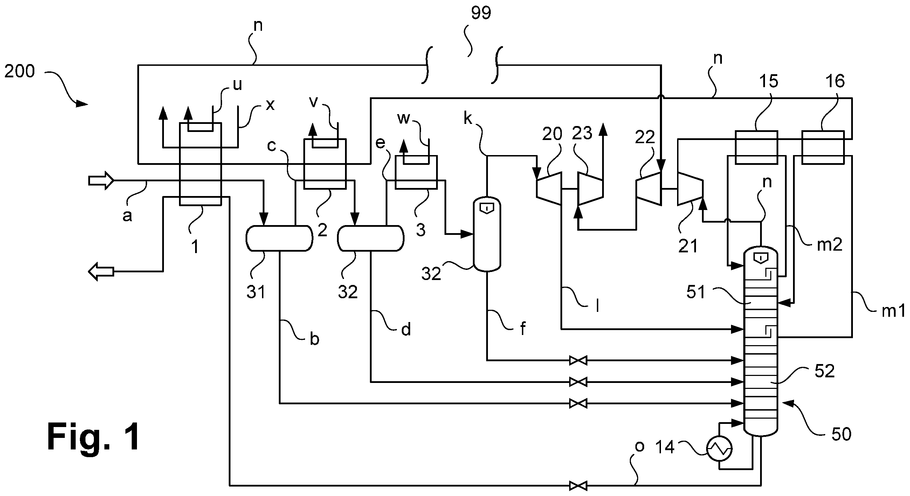

Herkömmliche Verfahren, beispielsweise ein Verfahren, wie es in Figur 1 veranschaulicht ist, umfassen, einen C2minus-Strom unter Druck stufenweise in Wärmetauschern abzukühlen und stromab dieser Wärmetauscher jeweils flüssige Kondensate abzuscheiden. Ein bei einem Druck von ca. 30 bar abs. und einer Temperatur von unter ca. -100°C gasförmig verbleibe nder Anteil des ursprünglich eingespeisten C2minus-Stroms wird in einem Expander entspannt. Der in dem Expander entspannte Anteil und die zuvor aus dem C2minus-Strom abgeschiedenen Kondensate werden in unterschiedlichen Höhen in eine Rektifikationskolonne (sogenannter Demethanizer) eingespeist.

Im Sumpf der Rektifikationskolonne wird ein flüssiges Sumpfprodukt , das im Wesentlichen Kohlenwasserstoffe mit zwei Kohlenwasserstoffen aufweist, gebildet und abgezogen. Ein gasförmiges Kopfprodukt, das im Wesentlichen Methan und Wasserstoff enthält, wird vom Kopf der Rektifikationskolonne als Kopfstrom abgezogen und durch Entspannen in einem Expander ca. -160°C abgekühlt.

Der entspannte Kopfstrom wird zur Abkühlung und zumindest teilweisen Verflüssigung eines ersten gasförmigen Stoffstroms aus der Rektifikationskolonne in einem ersten Plattentauscher und eines zweiten gasförmigen Stoffstroms in einem zweiten Plattentauscher verwendet. Die verwendete Rektifikationskolonne ist zweiteilig ausgebildet und die jeweils zumindest teilweise verflüssigten gasförmigen Stoffströme werden als Rücklauf auf die beiden Teile der Rektifikationskolonne verwendet. Anschließend wird der entspannte Kopfstrom noch zur Abkühlung des C2minus-Stroms verwendet und damit bei der Bildung der Kondensate eingesetzt.

Nachteilig an dem erläuterten Verfahren ist, dass die erwähnten Plattentauscher, die zur Abkühlung und zumindest teilweisen Verflüssigung des ersten gasförmigen Stoffstroms und des zweiten gasförmigen Stoffstroms aus der Rektifikationskolonne verwendet werden, oberhalb der Rektifikationskolonne angeordnet werden müssen, damit diese Stoffströme bzw. die verflüssigten Anteile als Rücklauf auf die Rektifikationskolonne zurückfließen können. Der entspannte Kopfstrom mit einem aufgrund der Entspannung gewissen Flüssiganteil muss aufwendig zu diesen Plattentauschern hinauftransportiert werden. Die Verwendung dieses entspannten Kopfstroms als Kältemittel in den Plattentauschern in großer Höhe ist mit technischen Schwierigkeiten verbunden, da Flüssig- und Gasphase vorzugsweise separat in die Plattentauscher geführt werden und ein geringer Druckverlust toleriert werden kann.

Die Installation der Plattentauscher am Kopf der Rektifikationskolonne ist zusammengefasst aufwendig und kostspielig. Entsprechendes gilt auch für eine vergleichbare Demethanisierung eines Komponentengemischs, das neben Kohlenwasserstoffe mit zwei Kohlenstoffatomen zusätzlich schwerere Kohlenwasserstoffe enthält. Aufgabe der Erfindung ist es daher, diese Nachteile des Standes der Technik zu beheben.

### Offenbarung der Erfindung

Vor diesem Hintergrund schlägt die Erfindung ein Verfahren zur Trennung eines Komponentengemischs, welches im Wesentlichen Kohlenwasserstoffe mit zwei oder zwei und mehr Kohlenstoffatomen, Methan und Wasserstoff enthält, und eine entsprechende Trennvorrichtung mit den jeweiligen Merkmalen der unabhängigen Patentansprüche vor. Bevorzugte Ausgestaltungen sind Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Die vorliegende Erfindung kommt insbesondere zur Trennung von Komponentengemischen zum Einsatz, die durch Dampfspaltverfahren erhalten werden. Sie ist jedoch nicht auf solche beschränkt. Das im Wesentlichen Kohlenwasserstoffe mit zwei oder zwei und mehr Kohlenstoffatomen, Methan und Wasserstoff enthaltende Komponentengemisch wird, wenn es unter Einsatz eines Dampfspaltverfahrens hergestellt wird, als Fraktion eines in dem Dampfspaltverfahren gebildeten sogenannten Spaltgases gebildet. Hierzu wird ein entsprechendes Spaltgas typischerweise von Wasser, Sauergasen und öl- und benzinartigen Komponenten befreit und verdichtet, wie bereits erläutert. Es stellt dann das im Wesentlichen Kohlenwasserstoffe mit zwei und mehr Kohlenstoffatomen, Methan und Wasserstoff enthaltende Komponentengemisch dar. Das im Wesentlichen Kohlenwasserstoffe mit zwei Kohlenstoffatomen, Methan und Wasserstoff enthaltende Komponentengemisch kann wiederum hieraus durch Abtrennen von Kohlenwasserstoffen mit drei und ggf. mehr Kohlenstoffatomen, insbesondere mittels Rektifikation, gebildet werden. Entweder das im Wesentlichen Kohlenwasserstoffe mit zwei und mehr Kohlenstoffatomen, Methan und Wasserstoff enthaltende Komponentengemisch oder das im Wesentlichen Kohlenwasserstoffe mit zwei Kohlenstoffatomen, Methan und Wasserstoff enthaltende Komponentengemisch kann im Rahmen der vorliegenden Erfindung bearbeitet ("demethanisiert") werden.

Gängige Verfahren umfassen die Trennung des Spaltgases in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt für die "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Komponentengemische können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Komponentengemische können im hier verwendeten Sprachgebrauch ferner angereichert oder abgereichert an einer oder mehreren Komponenten sein, wobei sich diese Begriffe auf einen Gehalt in einem anderen Komponentengemisch beziehen, aus dem das betrachtete Komponentengemisch erhalten wurde. Das Komponentengemisch ist "angereichert", wenn es zumindest den 1,1-fachen, 1,5-fachen, 2-fachen, 5-fachen, 10-fachen, 100-fachen oder 1.000-fachen Gehalt, "abgereichert" wenn es höchstens den 0,9-fachen, 0,5-fachen, 0,1-fachen, 0,01-fachen oder 0,001-fachen Gehalt einer Komponente, bezogen auf das andere Komponentengemisch, enthält. Ist hier davon die Rede, dass ein Komponentengemisch "im Wesentlichen" eine oder mehrere Komponenten enthält, wird hierunter insbesondere verstanden, dass das Komponentengemisch zumindest reich an der einen oder den mehreren Komponenten im oben erläuterten Sinn ist oder ausschließlich die eine oder die mehreren Komponenten aufweist.

Ein Komponentengemisch ist von einem anderen Komponentengemisch "abgeleitet", wenn es zumindest einige in dem anderen Komponentengemisch enthaltene oder aus diesem erhaltene Komponenten aufweist. Ein in diesem Sinne abgeleitetes Komponentengemisch kann aus einem anderen Komponentengemisch durch Abtrennen oder Abzweigen einer Teilmenge oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen nicht in Form exakter Druck- bzw. Temperaturwerte verwendet werden müssen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können in disjunkten Bereichen liegen oder in Bereichen, die einander überlappen. Insbesondere schließen dabei Druckniveaus geringfügig unterschiedliche Drücke, die sich aufgrund von unvermeidlichen oder zu erwartenden Druckverlusten ergeben, ein. Entsprechendes gilt für Temperaturniveaus. Bei den hier in bar angegebenen Druckniveaus handelt es sich um Absolutdrücke.

Ein "Wärmetauscher" dient zur indirekten Übertragung von Wärme zwischen zumindest zwei z.B. im Gegenstrom zueinander geführten Stoffströmen, beispielsweise einem wärmeren gasförmigen Stoffstrom und einem oder mehreren kälteren flüssigen Stoffströmen. Ein Wärmetauscher kann aus einem einzelnen oder mehreren parallel und/oder seriell verbundenen Wärmetauscherabschnitten gebildet sein, z.B. aus einem oder mehreren Plattenwärmetauscherblöcken. Ein Wärmetauscher weist "Passagen" auf, die als getrennte Fluidkanäle mit Wärmeaustauschflächen ausgebildet sind.

Ein "Flüssigkeitsabscheider" oder "Abscheidebehälter" ist ein Behälter, in dem aus einem gasförmigen Stoffstrom oder einem Zweiphasenstrom (der zum Teil flüssig und zum Teil gasförmig vorliegt), eine Flüssigkeit, sogenanntes Kondensat, abgeschieden wird. Das Kondensat kann aus dem Flüssigkeitsabscheider (typischerweise aus einem oberen Bereich, "Kopf") zumindest teilweise gasförmig entnommen werden.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemisch mit flüssigen und gasförmigen Anteilen, ggf. auch im überkritischen Zustand, bereitgestelltes Komponentengemisch (Fluid) zumindest teilweise aufzutrennen, also aus dem Komponentengemisch jeweils Reinstoffe oder Komponentengemische zu erzeugen, die gegenüber dem Komponentengemisch bezüglich zumindest einer Komponente angereichert bzw. abgereichert oder reich bzw. arm im oben erläuterten Sinne sind. Rektifikationskolonnen sind aus dem Bereich der Trenntechnik hinlänglich bekannt. Typischerweise sind Rektifikationskolonnen als zylindrische Metallbehälter ausgebildet, die mit Einbauten, beispielsweise Siebböden oder geordneten und ungeordneten Packungen, ausgerüstet sind. Eine Rektifikationskolonne zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Rektifikationskolonne mittels eines Sumpfverdampfers erwärmt, so dass kontinuierlich ein Teil der Sumpfflüssigkeit verdampft und in der Rektifikationskolonne gasförmig aufsteigt. Eine Rektifikationskolonne ist ferner typischerweise mit einem sogenannten Kopfkondensator versehen, in den zumindest ein Teil eines sich in einem oberen Bereich der Rektifikationskolonne anreichernden Gasgemischs oder ein entsprechendes Reingas, hier als Kopfgas bezeichnet, eingespeist zu einem Teil zu einem Kondensat verflüssigt und als flüssiger Rücklauf am Kopf der Rektifikationskolonne aufgegeben wird. Ein Teil des aus dem Kopfgas erthaltenen Kondensats kann anderweitig verwendet werden. Zur Auslegung und Ausgestaltung von Rektifikationskolonnen sei auf einschlägige Lehrbücher verwiesen (siehe beispielsweise Sattler, K: Thermische Trennverfahren: Grundlagen, Auslegung, Apparate, 3. Auflage 2001, Weinheim; Wiley-VCH).

Bei einer "Destillationseinrichtung" handelt es sich im hier verwendeten Sprachgebrauch um eine Trenneinrichtung, welche mindestens zwei verschiedenen Destillationseinheiten aufweist. Eine Destillationseinheit kann beispielsweise eine Rektifikationskolonne sein oder Komponenten, wie sie für eine Rektifikationskolonne typisch sind, umfassen. Eine Destillationseinrichtung kann auch in Form einer Rektifikationskolonne ausgebildet sein, die zwei Abschnitte aufweist, die über einen Boden voneinander getrennt sind, wobei sich auf dem Boden Flüssigkeit anstaut, welche über ein Wehr in den darunter liegenden Abschnitt abfließt und damit einen Rücklauf auf den darunter liegenden Abschnitt bildet. Auch ein Flüssigkeitsaustausch über Leitungen kann erfolgen.

### Vorteile der Erfindung

Die vorliegende Erfindung geht von einem insoweit bekannten Verfahren zur Trennung eines Komponentengemischs aus, das im Wesentlichen Kohlenwasserstoffe mit zwei oder zwei und mehr Kohlenstoffatomen, sowie Methan und Wasserstoff aufweist. Wie zuvor erläutert kann ein derartiges Komponentengemisch als aufbereitetes, aber noch nicht weiter trenntechnisch bearbeitetes Spaltgas, aber auch als C2minus-Fraktion vorliegen. Ein derartiges Verfahren wird unter Verwendung einer Destillationseinrichtung durchgeführt, in der ein Methan und Wasserstoff enthaltender Stoffstrom von einem schwereren Anteil getrennt wird, also ein Komponentengemisch, das im Wesentlichen Kohlenwasserstoffe mit zwei oder zwei und mehr Kohlenstoffatomen aufweist, und ein Komponentengemisch, das im Wesentlichen Methan und Wasserstoff aufweist, gebildet werden.

Bei einem derartigen Verfahren wird Fluid des Komponentengemischs auf einem ersten Druckniveau stufenweise von einem ersten Temperaturniveau über zwei oder mehrere Zwischentemperaturniveaus auf ein zweites Temperaturniveau abgekühlt. Auf jedem der Zwischentemperaturniveaus werden dabei auf dem ersten Druckniveau, d.h. jeweils ohne Entspannung, Kondensate aus dem Fluid abgeschieden.
Wird hier angegeben, dass "Fluid eines Stoffstroms" oder "Fluid eines Komponentengemischs" in irgendeiner Weise behandelt wird, sei hierunter verstanden, dass beispielsweise das gesamte Fluid, ein von einem Ausgangsfluid abgeleitetes Fluid oder ein Teilstrom eines Stoffstroms, der aus einem entsprechenden Fluid gebildet wurde, verwendet wird. Insbesondere werden in dem zuvor erläuterten Verfahrensschritt jeweils ein Kondensat und ein gasförmig verbleibender Anteil aus einem entsprechenden Fluid gebildet. Der gasförmig verbleibende Anteil wird dabei jeweils auf ein nächsttieferes Temperaturniveau (also die Zwischentemperaturniveaus oder letztlich das zweite Temperaturniveau) abgekühlt.

Fluid des Komponentengemischs, das auf dem zweiten Temperaturniveau gasförmig verbleibt, also das Fluid, das nicht in Form derjeweiligen Kondensate abgeschieden wird, wird auf dem ersten Druckniveau in einen Expander eingespeist und auf ein zweites Druckniveau entspannt, wobei durch die Entspannung eine Abkühlung sowie eine Teilkondensation erfolgt und somit ein Zweiphasenstrom gebildet wird.
Die durch das Abkühlen auf die Zwischentemperaturniveaus und das zweite Temperaturniveau gebildeten Kondensate sowie der Zweiphasenstrom werden jeweils zumindest teilweise in eine Destillationseinrichtung eingespeist, die auf dem zweiten Druckniveau betrieben wird, wobei in der Destillationseinrichtung zumindest ein flüssiger Stoffstrom, der im Wesentlichen Kohlenwasserstoffe mit zwei oder zwei und mehr Kohlenstoffatomen aufweist, und ein gasförmiger Stoffstrom, der im Wesentlichen Methan und Wasserstoff aufweist, gewonnen und aus der Destillationseinrichtung abgezogen werden.

In dem Verfahren werden Fluid der durch das Abkühlen auf die Zwischentemperaturniveaus und das zweite Temperaturniveau gebildeten Kondensate und des Zweiphasenstroms jeweils von dem ersten Druckniveau auf ein zweites Druckniveau unterhalb des ersten Druckniveaus entspannt und in die Destillationseinrichtung eingespeist, die auf dem zweiten Druckniveau betrieben wird.

Erfindungsgemäß liegt das erwähnte zweite Temperaturniveau bei -125 bis -150 °C, insbesondere bei ca. -145 °C. Dieses liegt deutlich unter entsprechenden Temperaturniveaus, wie sie in Verfahren gemäß dem Stand der Technik bei der Abkühlung entsprechender Einsatzgemische verwendet werden. Auf das Beispiel der Figur 1 wird verwiesen. Aufgrund der anschließenden Entspannung verringert sich das Temperaturniveau dabei nochmals deutlich, in dem Beispiel von ca. -145 °C insbesondere auf ca. -162 °C.

Die Destillationseinrichtung umfasst erfindungsgemäß eine erste Destillationseinheit und eine zweite Destillationseinheit, wobei die erste Destillationseinheit auf einem dritten Temperaturniveau unterhalb des zweiten Temperaturniveaus betrieben wird und die zweite Destillationseinheit auf einem vierten Temperaturniveau oberhalb des zweiten Temperaturniveaus betrieben wird. Das Fluid des Zweiphasenstroms wird dabei in die ersteDestillationseinheit eingespeist. Der flüssige Stoffstrom, der im Wesentlichen Kohlenwasserstoffe mit zwei oder zwei und mehr Kohlenstoffatomen aufweist, wird aus dem Sumpf der zweiten Destillationseinheit und der gasförmige Stoffstrom, der im Wesentlichen Methan und Wasserstoff aufweist, am Kopf der ersten Destillationseinheit abgezogen.

Das dritte Temperaturniveau unterhalb des zweiten Temperaturniveaus in der ersten Destillationseinheit ergibt sich insbesondere durch die Einspeisung des auf das zweite Temperaturniveau abgekühlten und anschließend entspannten Fluids, also des Zweiphasenstroms. Vorteilhafterweise wird der ersten Destillationseinheit dabei kein gasförmiges Fluid entnommen, das verflüssigt als Rücklauf auf die erste Destillationseinheit zurückgeführt wird, sondern sämtliches flüssig in die erste Destillationseinheit eingespeistes Fluid wird ausschließlich in Form der durch das Abkühlen auf die Zwischentemperaturniveaus und das zweite Temperaturniveau gebildeten Kondensate und/oder eines flüssigen Anteils des Zweiphasenstroms bzw. jeweils Teilen hiervon bereitgestellt.

Durch den Betrieb der ersten Destillationseinheit auf dem dritten Temperaturniveau und die Einspeisung insbesondere des Zweiphasenstroms in die erste Destillationseinheit kann dieser ein im Wesentlichen an Kohlenwasserstoffen mit zwei Kohlenstoffatomen freier Kopfstrom, nämlich der bereits erwähnte gasförmige Stoffstrom, der im Wesentlichen Methan und Wasserstoff aufweist, gewonnen werden, ohne dass hierzu ein Rückfluss aus Kopfgas mittels separater und insbesondere über dem Kopf der Rektifikationskolonne angeordneter Wärmetauscher gebildet werden muss wie im Stand der Technik.

Das vierte Temperaturniveau oberhalb des zweiten Temperaturniveaus in der zweiten Destillationseinheit wird insbesondere durch die Temperaturen der in die Destillationseinrichtung eingespeisten Kondensate erzielt, die durch das Abkühlen auf die Zwischentemperaturniveaus und das zweite Temperaturniveau gewonnen werden. Durch den Betrieb auf einem entsprechend höheren Temperaturniveau, das auch oberhalb eines entsprechenden Temperaturniveaus gemäß dem Stand der Technik liegt, kann ein Rücklauf auf die zweite Destillationseinheit gebildet werden, ohne dass hierzu eine starke Abkühlung wie im Stand der Technik erforderlich ist. Vielmehr kann hierzu auf bekannte Kältemittel wie Ethylen zurückgegriffen werden. Ein Ethylenkältekreislauf ist in einer entsprechenden Anlage ohnehin zur Abkühlung des Einsatzgemischs vorhanden, wie bereits erläutert.

Im Rahmen der vorliegenden Erfindung wird damit wie im Stand der Technik auch ein rückzuführender gasförmiger Stoffstrom aus der Destillationseinrichtung, jedoch nur der erwähnten zweiten Destillationseinheit, entnommen, in einen Kondensator eingespeist, im Kondensator abgekühlt und verflüssigt, und auf die Destillationseinrichtung bzw. deren zweite Destillationseinheit als Rücklauf aufgegeben. Der Kondensator wird jedoch auf einemTemperaturniveau oberhalb des zweiten Temperaturniveaus betrieben. Die Destillationseinrichtung weist also vorteilhafterweise einen Kondensator auf, der auf einem derartigen Temperaturniveau betrieben wird.

Durch den Einsatz der erfindungsgemäßen Maßnahmen wird vermieden, dass, wie im Stand der Technik, ein erster gasförmiger Stoffstrom aus der Rektifikationskolonne in einem ersten Plattentauscher und ein zweiter gasförmiger Stoffstrom in einem zweiten Wärmetauscher auf sehr niedrige Temperaturen abgekühlt und verflüssigt werden müssen, um auf die Rektifikationskolonne einen Rücklauf bereitstellen zu können. Im Gegensatz zu dem bekannten Verfahren ermöglicht die vorliegende Erfindung also das Einsparen der zwei Plattentauscher am Kopf der Rektifikationskolonne, in denen herkömmlicherweise nahezu die gesamte C1minus-Fraktion als Kältemittel verwendet wird und welche aufwendig zu den Plattentauschern transportiert werden muss.

Erfindungsgemäß liegt das erwähnte dritte Temperaturniveau bei -150 bis -170 °C, insbesondere bei ca. -162 °C. Dieses liegt deutlich unter Temperaturniveaus, wie sie in Verfahren gemäß dem Stand der Technik bei der Abkühlung entsprechender Einsatzgemische verwendet werden. Auf das Beispiel der Figur 1 wird verwiesen. Das erwähnte vierte Temperaturniveau liegt bei -50 bis -130 °C. Entsprechendes gilt für das Temperaturniveau in dem erwähnten Kondensator. Das erste Druckniveau liegt insbesondere bei 25 bis 30 bar, das zweite Druckniveau bei 10 bis 15 bar.

Der Einsatz der erfindungsgemäßen Lösung verändert die Stoff- und Wärmebilanz eines entsprechenden Systems praktisch nicht und zieht keine weitere Beeinflussung des Gesamtverfahrens nach sich. Die Erfindung ermöglicht eine Reduktion der Investitionskosten und eine Vereinfachung bei der Installation der Anlage.

Vorteilhafterweise wird Fluid des aus der Destillationseinrichtung abgezogenen gasförmigen Stoffstroms, der im Wesentlichen Methan und Wasserstoff aufweist, also Kopfgas der Destillationseinrichtung bzw. der ersten Destillationseinheit der Destillationseinrichtung, zumindest zum Abkühlen des Fluids des Kohlenstoffgemischs von dem ersten Temperaturniveau über die Zwischentemperaturniveaus auf das zweite Temperaturniveau verwendet. Dies ermöglicht eine effiziente Abkühlung entsprechenden Fluids. Hierbei kann insbesondere eine weitere Entspannung erfolgen, um die erforderlichen tiefen Temperaturen zu erzeugen. Der aus der Destillationseinrichtung abgezogene gasförmige Stoffstrom wird dieser beispielsweise bei -150 bis -160 °C, insbesondere ca. -153 °C entn ommen. Ein entsprechendes Temperaturniveau wird hier auch als fünftes Temperaturniveau bezeichnet.

Eines der Zwischentemperaturniveaus liegt vorteilhafterweise bei -120 bis -125 °C und eines der Zwischentemperaturniveaus bei -140 bis -145 °C. Das kalte Kopfgas auf dem fünften Temperaturniveau wird vorteilhafterweise dazu verwendet, einen bei einer stromaufwärtigen Kondensation gasförmig gebliebenen Anteil des Komponentengemischs von dem Zwischentemperaturniveau bei -120 bis -125 °C auf das Zwischentemperaturniveau bei -140 bis -145 °C abzukühlen, wobei sich das Kopfgas auf ein Temperaturniveau von -120 bis -130 °C, beispielsweise ca. -126 °C erwärmt. Anschließend wird das Kopfgas vorteilhafterweise in einem Expander auf ein Temperaturniveau von -150 bis -160 °C, beispielswei se ca. -157 °C abgekühlt und zur Abkühlung jeweils bei stromaufwärtigen Kondensationen gasförmig gebliebener Anteile des Komponentengemischs auf Zwischentemperaturniveaus bei beispielsweise -120 bis -125 °C, -95 bis -100 °C,-74 bis -79 °C so wie -48 bis -53 °C verwendet. Zusätzlich wird Ethylenkältemittel verwendet, um eine entsprechende Abkühlung auf die Zwischentemperaturniveaus vorzunehmen.

Vorteilhafterweise wird die Sumpfflüssigkeit der ersten Trenneinheit gesammelt und auf die zweite Trenneinheit geleitet, um in der zweiten Trenneinheit als Rücklauf bzw. als Kühlung zu dienen.

Das erfindungsgemäße Verfahren eignet sich in besonderer Weise zur Trennung eines Komponentengemischs, das aus einem mittels eines Dampfspaltprozess gewonnenen Spaltgas erhalten wird.

Eine entsprechende Trenneinrichtung ist ebenfalls Gegenstand der Erfindung. Die Trenneinrichtung ist zur Trennung eines im Wesentlichen Kohlenwasserstoffe mit zwei oder zwei und mehr Kohlenstoffatomen sowie Methan und Wasserstoff aufweisenden Komponentengemischs eingerichtet und weist eine Destillationseinrichtung, einen Kondensator und mindestens einen Expander auf. Zu den weiteren Bestandteilen einer entsprechenden Anlage sei auf die obigen Erläuterungen verwiesen.

Die Erfindung und Ausführungsformen der Erfindung werden unter Bezugnahme auf die beigefügten Zeichnungen näher erläutert.

### Kurze Beschreibung der Zeichnungen

Figur 1 zeigt eine Trenneinrichtung zur Trennung eines Komponentengemischs gemäß Stand der Technik.
Figur 2 zeigt eine Trenneinrichtung zur Trennung eines Komponentengemischs gemäß einer Ausführungsform der Erfindung.
Figur 3 zeigt eine Trenneinrichtung zur Trennung eines Komponentengemischs gemäß einer zweiten Ausführungsform der Erfindung.
Figur 4 zeigt eine Trenneinrichtung zur Trennung eines Komponentengemischs gemäß einer dritten Ausführungsform der Erfindung.

### Ausführliche Beschreibung der Zeichnungen

In den Figuren sind einander entsprechende Elemente mit identischen Bezugszeichen angegeben und werden der Übersichtlichkeit halber nicht wiederholt erläutert. In den nachfolgenden Figuren wird die Erfindung unter Bezugnahme auf eine trenntechnische Behandlung eines im Wesentlichen Kohlenwasserstoffe mit zwei Kohlenstoffatomen sowie Methan und Wasserstoff aufweisenden Komponentengemischs beschrieben. Sie eignet sich, wie erwähnt, aber in gleicher Weise zur trenntechnischen Behandlung eines im Wesentlichen Kohlenwasserstoffe mit zwei und mehr Kohlenstoffatomen sowie Methan und Wasserstoff aufweisenden Komponentengemischs.
Figur 1 zeigt eine Trenneinrichtung zur Trennung eines Komponentengemischs gemäß einer nicht erfindungsgemäßen Ausführungsform. Die Trenneinrichtung ist insgesamt mit 200 bezeichnet und zur Trennung eines Komponentengemischs, das im Wesentlichen Kohlenwasserstoffe mit zwei Kohlenstoffatomen, Methan und Wasserstoff aufweist (also einer C2minus-Fraktion) eingerichtet. Die C2minus-Fraktion wird der Trenneinrichtung 200 in Form eines Stoffstroms a zugeführt.

Die Trenneinrichtung 200 umfasst einen ersten Wärmetauscher 1, einen zweiten Wärmetauscher 2 und einen dritten Wärmetauscher 3. Der Stoffstrom a wird zunächst durch den ersten Wärmetauscher 1 geführt und in diesem abgekühlt. Er wird anschließend in einen ersten Flüssigkeitsabscheider 31 eingespeist. Die Abkühlung in dem ersten Wärmetauscher 1 erfolgt derart, dass sich in dem ersten Flüssigkeitsabscheider 31 ein flüssiges Kondensat abscheidet. Dieses wird am Boden des ersten Flüssigkeitsabscheiders 31 als Stoffstrom b abgezogen. Die weitere Verwendung des Stoffstroms b wird unten erläutert.

Ein in dem ersten Flüssigkeitsabscheider 31 gasförmig verbliebener Anteil des Stoffstroms a wird als Stoffstrom c durch den zweiten Wärmetauscher 2 geführt und anschließend in einen zweiten Flüssigkeitsabscheider 32 eingespeist. Auch in diesem scheidet sich ein flüssiges Kondensat am Boden ab und wird in Form des Stoffstroms d abgezogen. Ein noch immer gasförmig verbliebener Anteil des Stoffstroms c wird als Stoffstrom e durch einen dritten Wärmetauscher 3 geführt und anschließend in einen dritten Flüssigkeitsabscheider 33 eingespeist. Auch in diesem scheidet sich ein flüssiges Kondensat am Boden ab und wird in Form des Stoffstroms f abgezogen. Ein noch immer gasförmig verbliebener Anteil des Stoffstroms e wird als Stoffstrom k in einen Expander 20 geführt, entspannt, und zumindest teilweise verflüssigt. Ein auf diese Weise gebildeter Zweiphasenstrom wird in Form eines Stoffstroms I bereitgestellt.

Die Trenneinrichtung 200 umfasst ferner eine Rektifikationskolonne 50, die mit einem nicht näher erläuterten Sumpfverdampfer 14 betrieben wird, dessen Wärmetauscher beispielsweise mit einem aus anderen Anlagenteilen stammenden Propylenstrom betrieben wird. Der Rektifikationskolonne 50 sind ferner zwei Plattentauscher 15, 16 zugeordnet, deren Betrieb unten erläutert wird. Die Rektifikationskolonne 50 weist zwei Abschnitte 51, 52 auf.

Aufgrund der sukzessiven Abkühlung der Stoffströme a, c, e und k, weisen die entsprechend erhaltenen Kondensate, die in Form der Stoffströme b, d, f, l gewonnen werden, unterschiedliche Gehalte an Kohlenwasserstoffen mit zwei Kohlenstoffatomen, Methan und Wasserstoff auf. Insbesondere weist der Stoffstrom I einen höheren Methan- und Wasserstoffgehalt als der Stoffstrom f, der Stoffstrom f einen höheren Methan- und Wasserstoffgehalt als der Stoffstrom d, und der Stoffstrom d einen höheren Methan- und Wasserstoffgehalt als Stoffstrom b auf.

Die Stoffströme b, d, f und I werden daher in unterschiedlichen Höhen in die Rektifikationskolonne 50 eingespeist, die hierzu geeignete Aufgabeeinrichtungen zwischen den hier stark schematisiert dargestellten Böden aufweist.

Vom Kopf der Rektifikationskolonne 50 wird ein gasförmiger Stoffstrom n abgezogen und in einem Expander 21 entspannt und dadurch stark abgekühlt bzw. zumindest teilweise verflüssigt. Der Stoffstrom n enthält überwiegend Methan und Wasserstoff (es handelt sich also um eine sogenannte C1 minus-Fraktion). Der verflüssigte Stoffstrom n wird durch die beiden Plattentauscher 15, 16 geführt und dort zur Kühlung verwendet. Anschließend wird der Stoffstrom n durch den Wärmetauscher 2 geführt und zur Kühlung des gasförmigen Stoffstroms c verwendet sowie durch den Wärmetauscher 1 geführt und zur Kühlung des gasförmigen Stoffstroms a verwendet. Anschließend wird der Stoffstrom n, insbesondere nach einer Verwendung in einer Vorkühleinheit 99 durch zwei mit den Expandern 20 und 21 gekoppelte Booster 22, 23 geführt und als Tailgas aus der Anlage ausgeleitet.

Im Sumpf der Rektifikationskolonne 50 scheidet sich ein flüssiges Kondensat ab, das im Wesentlichen aus Kohlenwasserstoffen mit zwei Kohlenstoffatomen aufweist (es handelt sich also um eine sogenannte C2-Fraktion). Das Kondensat wird in Form des Stoffstroms o abgezogen, in dem ersten Wärmetauscher 1 erwärmt und beispielsweise anschließend einer weiteren Trenneinrichtung wie einem C2-Splitter zugeführt.

Ein gasförmiger Stoffstrom m1 wird gasförmig aus der Rektifikationskolonne 50 an einer ersten Position entnommen, im Plattentauscher 16 abgekühlt und dadurch zumindest teilweise verflüssigt und der Rektifikationskolonne 50 als Rücklauf an einer zweiten Position zugeführt, also per Schwerkraft zurück in die Rektifikationskolonne 50 geleitet. Ein weiterer gasförmiger Stoffstrom m2 wird aus der Rektifikationskolonne 50 an einer dritten Position gasförmig entnommen, im Plattentauscher 15 abgekühlt und dadurch zumindest teilweise verflüssigt und der Rektifikationskolonne 50 als Rücklauf an einer vierten Position zugeführt, also per Schwerkraft zurück in die Rektifikationskolonne 50 geleitet, wobei die erste Position sich unterhalb der zweiten Position, die zweite Position sich unterhalb der dritten Position und die dritte Position sich unterhalb der vierten Position befindet. Wie oben erwähnt wird in den Plattentauschern 15, 16 der verflüssigte Stoffstrom n zur Kühlung der Stoffströme m1 und m2 verwendet, der aufwendig zu den Plattentauschern, welche sich in großer Höhe befinden, transportiert werden muss. Die Installation dieser Plattentauscher 15, 16 am Kopf der Rektifikationskolonne 50 ist aufwendig und kostspielig.

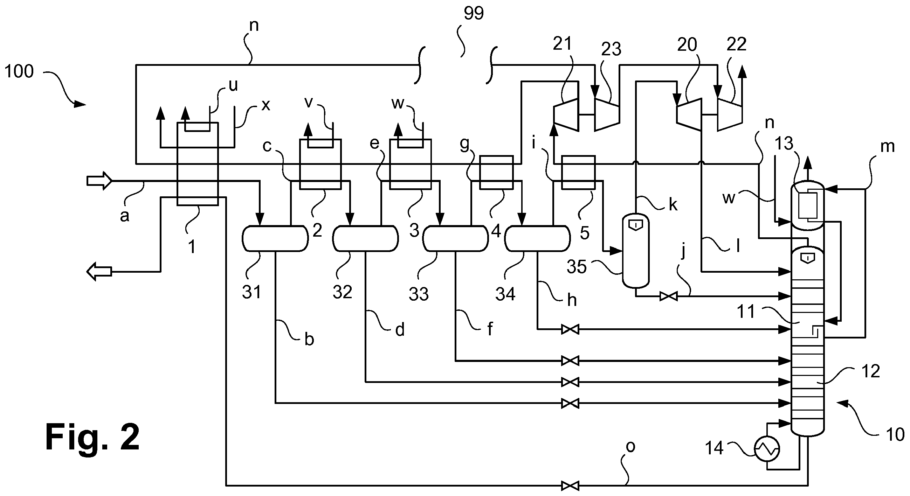

Figur 2 zeigt eine Trenneinrichtung 100 gemäß einer Ausführungsform der Erfindung. Die Trenneinrichtung 100 umfasst die wesentlichen Komponenten der in Figur 1 dargestellten Trenneinrichtung 200. Diese werden nicht erneut erläutert.

Auch in der Trenneinrichtung 100 ist eine Rektifikationskolonne mit zwei Abschnitten vorgesehen. Diese wird nachfolgend als Destillationseinrichtung 10 bezeichnet. Ihre zwei Abschnitte werden nachfolgend als erste Destillationseinheit 11 und zweite Destillationseinheit 12 bezeichnet. Der Trenneinrichtung ist ein Kondensator 13 zugeordnet. In der Trenneinrichtung 100 ist die erste Destillationseinheit 11 oberhalb der zweiten Destillationseinheit 12 angeordnet. Der Kondensator 13 ist zudem oberhalb der ersten Destillationseinheit 11 angeordnet. Abweichend zu der in Figur 1 dargestellten Trenneinrichtung 200 wurde hier jedoch auf die Plattentauscher 15, 16 verzichtet. Ein dem Stoffstrom m1 in der Trenneinrichtung 100 entsprechender Stoffstrom m wird in dem Kondensator 13 mittels Ethylenkältemittel gekühlt und dabei zumindest teilweise verflüssigt. Der gasförmige Stoffstrom m wird dabei aus dem oberen Teil der zweiten Destillationseinheit 12 entnommen und in den unteren Teil der ersten Destillationseinheit 11 eingespeist. Da die erste Destillationseinheit 11 und die zweite Destillationseinheit 12 mittels eines Flüssigkeitsstaubodens voneinander getrennt sind, dient der rückgeführte Stoffstrom m zur Bereitstellung eines Rücklaufs auf die zweite Destillationseinheit 12. Ein dem Stoffstrom m2 in der Trenneinrichtung 100 entsprechender Stoffstrom ist hier nicht vorhanden. Ein Rücklauf auf die erste Destillationseinheit 11 wird stattdessen wie nachfolgend erläutert bereitgestellt.

Die sukzessive Abkühlung des C2minus-Stroms a wird um einen Wärmetauscher 4, zur Kühlung eines gasförmigen Stoffstroms g, und um einen Wärmetauscher 5, zur Kühlung eines gasförmigen Stoffstroms i, erweitert. Zusätzlich zu den Kondensaten bzw. Stoffströmen b, d, f und I werden Kondensate bzw. Stoffströme h und j in weiteren Flüssigkeitsabscheidern 34, 35 abgeschieden. Die jeweils gasförmig verbleibenden Anteile werden in Form der Stoffströme g und i durch die Wärmetauscher 4 und 5 geführt und auf die Zwischentemperaturniveaus bei -120 bis -125 °C (Strom g) bzw. - 140 bis -145 °C (Strom i) abgekühlt. Da der Stoffst rom j einen höheren Methan- und Wasserstoffanteil als der Stoffstrom h, und der Stoffstrom h einen höheren Methan- und Wasserstoffanteil als der Stoffstrom f aufweisen, werden diese in unterschiedlichen Höhen der Destillationseinrichtung 10 eingespeist.

Der Stoffstrom I in der Trenneinrichtung 200 wird vergleichbar gebildet wie der Stoffstrom I in der Trenneinrichtung 100, liegt jedoch aufgrund der weiteren Abkühlung in den Wärmetauschern 4 und 5 auf einer deutlich geringeren Temperatur vor. Aufgrund der weiteren Abkühlung können insbesondere die Stoffströme j und I als Rücklauf auf die erste Destillationseinheit 11 verwendet werden. Eine Verflüssigung eines Stoffstroms m2 wie in der Trenneinrichtung 100 ist daher nicht erforderlich, so dass, wie erwähnt, auf die Wärmetauscher 15, 16 verzichtet werden kann.

Da in der vorliegenden Erfindung der gasförmige Stoffstrom n, der am Kopf der Destillationseinrichtung entnommen wird, nicht zur Kühlung in den Plattentauschern 15, 16 verwendet werden muss, kann dieser zur Kühlung in den Wärmetauschern 1, 2, 3, 4, 5 verwendet werden. Auf diese Weise können die erwähnten tiefen Temperaturen erreicht werden.

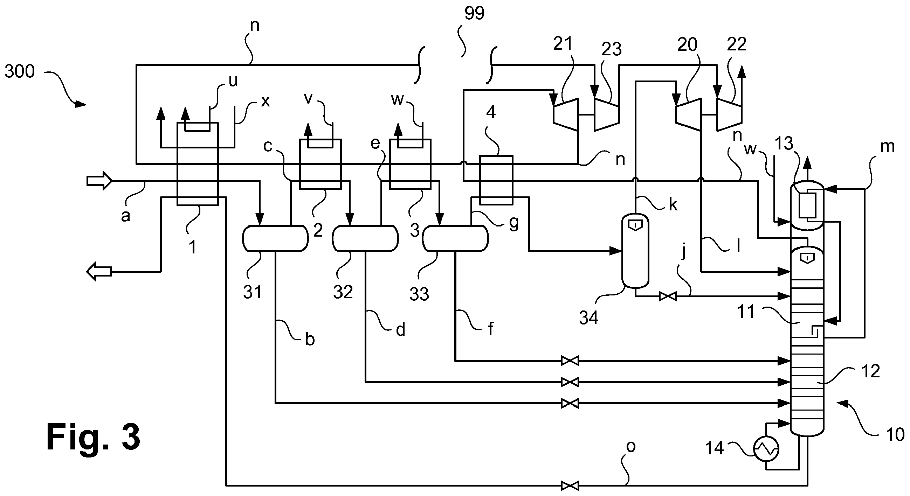

Figur 3 zeigt eine Trenneinrichtung 300 gemäß einer weiteren Ausführungsform der Erfindung. Hierbei wurde die Trenneinrichtung gemäß des Stands der Technik nur um einen Wärmetauscher 4 und einen Flüssigkeitsabscheider 34 erweitert.

In den Figuren 1 bis 3 werden die Wärmetauscher 1 bis 3 jeweils unter Verwendung weiterer Stoffströme u, v, w und x gekühlt. Bei den Stoffströmen u, v und w handelt es sich dabei um Ethylenkältemittel auf unterschiedlichen Druckniveaus, bei dem Stoffstrom x beispielsweise um einen rückgeführten Ethanstrom. Ein Ethylenkältemittelstrom w durch den Kondensator 13 ist entsprechend angegeben.

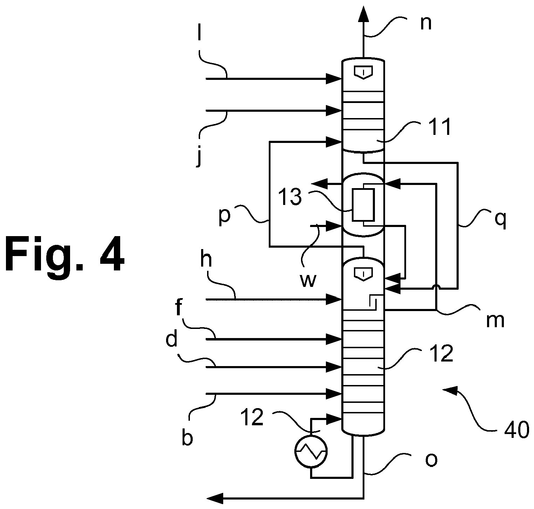

Figur 4 zeigt die Destillationseinheit 40 gemäß einer weiteren Ausführungsform der Erfindung. Hierbei ist der Kondensator 13 zwischen der ersten Destillationseinheit 11 und der zweiten Destillationseinheit 12 angeordnet, wobei die erste Destillationseinheit 11 oberhalb des Kondensators, und der Kondensator 13 oberhalb der zweiten Destillationseinheit 12 angeordnet ist. Ein Stoffaustausch erfolgt in Form eines Stoffstroms p und eines Stoffstroms q.

Weiterhin könnte der Kondensator 13 direkt in die Destillationseinheit 40 integriert werden, beispielsweise in Form eines Rohbündels oder eines Blocks zwischen der ersten Destillationseinheit 11 und der zweiten Destillationseinheit 12. Ensprechend würden einbauten wie der Flüssigkeitsverschluss entfallen.

In der folgenden Tabelle sind Temperaturen ausgewählter Stoffströme angegeben. Die Angaben in Klammern stellen jeweils bevorzugte Temperaturbereiche dar, der nach der Klammer angegebe Wert ist ein bevorzugtes Beispiel.

| **Trenneinrichtung** | **200** | **100** | **300** |
|---|---|---|---|
| Stoffstrom a vor Wärmetauscher 1 | (-25 bis -35 °C) | (-25 bis -35 °C) | (-25 bis -35 °C) |
| | -32 °C | -32 °C | -32 °C |
| Stoffstrom a nach Wärmetauscher 1 | (-45 bis -55 °C) | (-45 bis -55 °C) | (-45 bis -55 °C) |
| | -51 °C | -51 °C | -51 °C |
| Stoffstrom c nach Wärmetauscher 2 | (-70 bis -80 °C) | (-74 bis -80 °C) | (-74 bis -80 °C) |
| | -76 °C | -76 ^{c}C | -76 °C |
| Stoffstrom e nach Wärmetauscher 3 | (-95 bis -100 °C) | (-95 bis -100 °C) | (-95 bis -100 °C) |
| | -97 °C | -97 °C | -97 °C |
| Stoffstrom g nach Wärmetauscher 4 | | (-115 bis -125 °C) | (-135 bis -145 °C) |
| | | -122 °C | -143 °C |
| Stoffstrom i nach Wärmetauscher 5 | | (-135 bis -145 °C) | |
| | | -143 °C | |
| Stoffstrom I nach Expander 20 | (-112 bis -118 °C) | (-155 bis -165 °C) | (-155 bis -165 °C) |
| | -115 °C | -162 °C | -162 °C |
| Stoffstrom n nach Entnahme | (-150 bis -155 °C) | (-150 bis -155 °C) | (-150 bis -155 °C) |
| | -152 °C | -153 °C | -153 °C |
| Stoffstrom o nach Entnahme | (-35 bis -45 °C) | (-35 bis -45 °C) | (-35 bis -45 °C) |
| | -36 °C | -36 °C | -36 °C |

## Patentansprüche

1. Verfahren zur Trennung eines im Wesentlichen Kohlenwasserstoffe mit zwei oder zwei und mehr Kohlenstoffatomen, Methan und Wasserstoff enthaltenden Komponentengemischs (C2minus) unter Verwendung einer Destillationseinrichtung (10), bei dem
- Fluid (a, c, e, g, i) des Komponentengemischs (C2minus) auf einem ersten Druckniveau stufenweise von einem ersten Temperaturniveau über zwei oder mehrere Zwischentemperaturniveaus auf ein zweites Temperaturniveau abgekühlt wird, wobei auf jedem der Zwischentemperaturniveaus Kondensate (b, d, f, h, j) aus dem Fluid (a, c, e, g, i) abgeschieden werden,
- Fluid (k) des Komponentengemischs (C2minus), das auf dem zweiten Temperaturniveau gasförmig verbleibt, durch Entspannen von dem ersten Druckniveau auf ein zweites Druckniveau unterhalb des ersten Druckniveaus abgekühlt wird, wobei ein Zweiphasenstrom (I) gebildet wird,
- Fluid der Kondensate (b, d, f, h, j) und Fluid des Zweiphasenstroms (I) in die Destillationseinrichtung (10) eingespeist wird, die auf dem zweiten Druckniveau betrieben wird, wobei in der Destillationseinrichtung (10) zumindest ein flüssiger Stoffstrom (o), der im Wesentlichen Kohlenwasserstoffe mit zwei oder zwei und mehr Kohlenstoffatomen aufweist, und ein gasförmiger Stoffstrom (n), der im Wesentlichen Methan und Wasserstoff aufweist, gewonnen und aus der Destillationseinrichtung (10) abgezogen werden,
**dadurch gekennzeichnet, dass**
- das zweite Temperaturniveau bei -125 bis -150 °C liegt,
- die Destillationseinrichtung (10) eine erste Destillationseinheit (11) und eine zweite Destillationseinheit (12) aufweist, wobei die erste Destillationseinheit (11) auf einem dritten Temperaturniveau unterhalb des zweiten Temperaturniveaus betrieben wird und die zweite Destillationseinheit (12) auf einem vierten Temperaturniveau oberhalb des zweiten Temperaturniveau betrieben wird, und
- das Fluid des Zweiphasenstroms in die erste Destillationseinheit (11) eingespeist wird.

2. Verfahren nach Anspruch 1, wobei die Destillationseinrichtung (10) einen Kondensator (13) aufweist, der auf dem Temperaturniveau oberhalb des zweiten Temperaturniveaus betrieben wird.

3. Verfahren nach Anspruch 1 oder 2, wobei das Fluid der Kondensate (b, d, f, h, j) zumindest teilweise in die erste und in die zweite Destillationseinheit (11) der Destillationseinrichtung (10) eingespeist wird.

4. Verfahren nach einem der Ansprüche 2 oder 3, wobei ein gasförmiger Stoffstrom (m) aus der zweiten Destillationseinheit (12) der Destillationseinrichtung (10) abgezogen, in dem Kondensator (13) abgekühlt und zur Bereitstellung eines flüssigen Rücklaufs auf die zweite Destillationseinheit (12) verwendet wird.

5. Verfahren nach einem der vorstehenden Ansprüche, wobei der flüssige Stoffstrom (o) von dem zweiten Druckniveau auf ein Druckniveau unterhalb des zweiten Druckniveaus entspannt wird.

6. Verfahren nach einem der vorstehenden Ansprüche, wobei das Fluid (a, c, e, g, i) des Komponentengemischs (C2minus) unter Einsatz eines Ethan- und/oder Ethylenkältemittels auf unterschiedlichen Druckniveaus von dem ersten Temperaturniveau über die Zwischenniveaus auf das zweite Temperaturniveau abgekühlt wird.

7. Verfahren nach einem der vorstehenden Ansprüche, wobei die Zwischentemperaturniveaus ein Zwischentemperaturniveau bei -48 bis -53 °C und/oder ein Zwischentemperaturniveau bei -74 bis -79 °C und/oder ein Zwischentemperaturniveau bei -95 bis -100 °C und/od er ein Zwischentemperaturniveau bei -120 bis -125 °C und/o der ein Zwischentemperaturniveau bei -140 bis -145 °C umfas sen.

8. Verfahren nach einem der vorstehenden Ansprüche, wobei der gasförmige Stoffstrom (m) durch ein Ethylenkältemittel im Kondensator (13) abgekühlt wird.

9. Verfahren nach nach einem der vorstehenden Ansprüche, wobei der Kondensator (13) der Destillationseinrichtung (10) zwischen der ersten Destillationseinheit (11) und der zweiten Destillationseinheit (12) angeordnet ist.

10. Verfahren nach einem der vorstehenden Ansprüche, bei dem Fluid des gasförmigen Stoffstroms (n), der aus der Destillationseinrichtung (10) abgezogen wird, zumindest zum Abkühlen des Fluids (a, c, e, g, i) des Kohlenstoffgemischs (C2minus) von dem ersten Temperaturniveau über die Zwischentemperaturniveaus auf das zweite Temperaturniveau verwendet wird.

11. Verfahren nach einem der vorstehenden Ansprüche, wobei das Fluid des gasförmigen Stoffstroms (n) der aus der Destillationseinrichtung (10) abgezogen wird, in einem weiteren Expander (21) auf ein drittes Druckniveau unterhalb des zweiten Druckniveaus entspannt wird.

12. Verfahren nach einem der vorstehenden Ansprüche, wobei das Fluid des gasförmigen Stoffstroms (n) aus der Destillationseinrichtung (10) abgezogen wird, nach Verwendung zum Abkühlen des Fluids (a, c, e, g, i) des Kohlenstoffgemischs (C2minus) verdichtet wird, wobei zum Verdichten mit dem Expander (20, 21) gekoppelte Verdichter verwendet werden.

13. Verfahren nach nach einem der vorstehenden Ansprüche, das zur Trennung eines Komponentengemischs (C2minus) eingesetzt wird, das aus einem mittels eines Dampfspaltprozesses gewonnenen Spaltgases erhalten wird.

14. Trenneinrichtung (100), die zur Trennung eines im Wesentlichen Kohlenwasserstoffe mit zwei oder zwei und mehr Kohlenstoffatomen, Methan und Wasserstoff enthaltenden Komponentengemischs eingerichtet ist, wobei die Trenneinrichtung (100) dafür eingerichtet ist,
- Fluid (a, c, e, g, i,) des Komponentengemischs (C2minus) auf einem ersten Druckniveau stufenweise von einem ersten Temperaturniveau über zwei oder mehrere Zwischentemperaturniveaus auf ein zweites Temperaturniveau abzukühlen sowie auf jedem der Zwischentemperaturniveaus Kondensate (b, d, f, h, j) aus dem Fluid (a, c, e, g, i) abzuscheiden,
- Fluid (k) des Komponentengemischs (C2minus), das auf dem zweiten Temperaturniveau gasförmig verbleibt, durch Entspannen von dem ersten Druckniveau auf ein zweites Druckniveau unterhalb des ersten Druckniveaus abzukühlen, wobei ein Zweiphasenstrom (I) gebildet wird,
- Fluid der Kondensate (b, d, f, h, j) und Fluid des Zweiphasenstroms (I) in die Destillationseinrichtung (10) einzuspeisen, die Destillationseinrichtung (10) auf dem zweiten Druckniveau zu betreiben, und in der Destillationseinrichtung (10) zumindest einen flüssigen Stoffstrom (o), der im Wesentlichen aus Kohlenwasserstoffen mit zwei Kohlenstoffatomen aufweist, und einen gasförmigen Stoffstrom (n), der im Wesentlichen aus Methan und Wasserstoff aufweist, zu gewinnen und aus der Destillationseinrichtung (10) abzuziehen,
**dadurch gekennzeichnet, dass**
- die Destillationseinheit (10) in eine erste Destillationseinheit (11) und eine zweite Destillationseinheit (12) aufgeteilt ist, wobei Mittel vorgesehen sind, die dafür eingerichtet sind, die erste Destillationseinheit (11) auf einem dritten Temperaturniveau unterhalb des zweiten Temperaturniveaus zu betreiben und Mittel vorgesehen sind, die dafür eingerichtet sind, die zweite Destillationseinheit (12) auf einem vierten Temperaturniveau oberhalb des zweiten Temperaturniveaus zu betreiben, und
- die Destillationseinheit (10) einen Kondensator (13) aufweist, der auf dem vierten Temperaturniveau betrieben wird.

15. Trenneinrichtung (100) nach Anspruch 14, die zur Durchführung eines Verfahrens nach einem der Ansprüche 1 bis 13 eingerichtet ist.
